Europäisches Patentamt

European Patent Office

Office européen des brevets

(19)

(11) EP 0 780 368 B1

(12) **EUROPÄISCHE PATENTSCHRIFT**

(45) Veröffentlichungstag und Bekanntmachung des Hinweises auf die Patenterteilung:
**21.07.1999 Patentblatt 1999/29**

(51) Int. Cl.$^6$: **C07C 319/02**, C07C 323/52

(21) Anmeldenummer: 96119601.1

(22) Anmeldetag: **06.12.1996**

(54) **Verfahren zur Herstellung von Thioglykolsäure**

Process for the preparation of thioglycolic acid

Procédé de préparation de l'acide thioglycolique

(84) Benannte Vertragsstaaten:
**AT BE CH DE ES FR GB IT LI NL SE**

(30) Priorität: **21.12.1995 DE 19547867**

(43) Veröffentlichungstag der Anmeldung:
**25.06.1997 Patentblatt 1997/26**

(73) Patentinhaber: **Akzo Nobel N.V.**
**6824 BM Arnhem (NL)**

(72) Erfinder:
• **Bergfeld, Manfred Josef, Dr.**
**63906 Erlenbach-Mechenhard (DE)**
• **Blaufelder, Christian, Dr.**
**96450 Coburg (DE)**

(74) Vertreter: **Fett, Günter et al**
**Akzo Nobel GmbH**
**Kasinostrasse 19-21**
**42103 Wuppertal (DE)**

(56) Entgegenhaltungen:
**EP-A- 0 476 339         DE-A- 3 534 612**
**US-A- 4 082 790**

• **PATENT ABSTRACTS OF JAPAN vol. 009, no. 160 (C-289), 4.Juli 1985 & JP 60 036456 A (SANTEN SEIYAKU KK), 25.Februar 1985,**

Bemerkungen:
Die Akte enthält technische Angaben, die nach dem Eingang der Anmeldung eingereicht wurden und die nicht in dieser Patentschrift enthalten sind.

Anmerkung:    Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Herstellung von Thioglykolsäure aus Monochloressigsäure und Schwefelwasserstoff.

Thioglykolsäure, auch Mercaptoessigsäure genannt, ist eine Chemikalie, die vor allem in Form ihrer Salze und Ester vielseitige Verwendung gefunden hat. So werden Thioglykolate in Kaltwellpräparaten in der Haarbehandlung eingesetzt, sie finden u.a. Verwendung bei der Permanentverformung von Wollwaren; Isooctylthioglykolat dient zum Aufbau von zinnhaltigen Stabilisatoren für PVC, Thioglykolsäureester werden auch als Antioxydantien in der Kautschukindustrie eingesetzt.

Die Herstellung der Thioglykolsäure geschieht vorwiegend durch Umsetzung von Monochloressigsäure bzw. deren Salze mit Hydrogensulfiden wie Kalium- oder Natriumhydrogensulfid.

Man kann die Reaktion des Hydrogensulfids wie Natrium- oder Ammoniumhydrogensulfid mit der Monochloressigsäure auch in der Weise durchführen, daß man in eine wäßrige Lösung von Monochloressigsäure und Natrium- oder Ammoniumhydroxid Schwefelwasserstoff einleitet, wie z.B. in der DE-AS 2 354 098 beschrieben wird.

Von Nachteil bei derartigen Verfahren ist, daß nicht unerhebliche Mengen an unerwünschten Nebenprodukten entstehen und vor allem, daß entsprechend der folgenden Reaktionsgleichung Natriumchlorid bzw. Ammoniumchlorid als Abfallprodukt anfällt, das entsorgt werden muß.

$$\text{Cl-CH}_2\text{COONa} + \text{NaSH} \rightarrow \text{HS-CH}_2\text{COONa} + \text{NaCl} \qquad (1)$$

Auch das in der DE-OS 2 711 867 beschriebene Verfahren, bei dem diese Umsetzung unter einem hohen Kohlendioxid-Partialdruck stattfindet, kann, abgesehen davon, daß auch hier in nicht unbeachtlichem Maße Nebenprodukte entstehen, dem Nachteil der Bildung von Natriumchlorid nicht abhelfen.

Schließlich führt auch das in der DE-A 35 34 612 beschriebene Verfahren zur Herstellung von Thioglykolsäure, wobei Mono- und Dichloressigsäure oder deren Alkalimetallsalze mit einem (Erd-)Alkalimetallhydrogensulfid bei einem $\text{H}_2\text{S}$-Partialdruck von mindestens 17,2 bar umgesetzt werden, zur Bildung beträchtlicher Mengen von anorganischem Abfall in Gestalt von Natriumchlorid und Schwefel.

Es sind auch Verfahren bekanntgeworden, bei denen Chloressigsäure direkt mit Schwefelwasserstoff umgesetzt wird. So wird gemäß der SU-Patentschrift 740.761 Chloressigsäure mit Schwefelwasserstoff bei Temperaturen von 520 bis 620°C bei atmosphärischem Druck umgesetzt. Abgesehen davon, daß ein Arbeiten bei derart hohen Temperaturen nachteilig ist, läßt auch die Ausbeute mit nur 90 % zu wünschen übrig. Auch zeigten eigene Versuche, daß ein beachtlicher Anteil an nur schwer entfernbaren Nebenprodukten entsteht, wie z.B. Thiodiglykolsäure oder Dithiodiglykolsäure.

In der US-PS 4 082 790 wird ein Verfahren zur Herstellung von Mercaptanen beschrieben, bei dem ein organisches Chlorid oder Bromid mit einer Mischung von Schwefelwasserstoff und Ammoniak oder einem Amin unter autogenem Druck umgesetzt wird. Von Nachteil bei diesem Verfahren sind die vielfach sehr langen Reaktionszeiten, die von mehreren Stunden bis in die Größenordnung von 1 Tag dauern; zudem ist das Arbeiten in einem geschlossenen Autoklaven umständlich.

Der Aufzählung in Spalte 2 dieser Patentschrift ist zu entnehmen, daß man zahlreiche chlor- oder bromhaltige Substanzen umsetzen kann. Neben Alkylchloriden wie Methyl-, Äthylchlorid usw., halogenierten Äthern, Ketonen usw. werden auch eine Reihe von halogenierten Carbonsäuren, u.a. auch Chloressigsäure genannt.

Auch zahlreiche primäre Amine mit Methylamin, Butylamin usw., sekundäre Amine wie Dimethylamin, Dipropylamin usw. und tertiäre Amine wie Trimethylamin, Triäthylamin oder Äthyldimethylamin werden neben Ammoniak als Base bei der Reaktion empfohlen. Die Herstellung von Thioglykolsäure aus Chloressigsäure findet sich in den Beispielen nicht. Auch fehlt jeglicher Hinweis, unter welchen Bedingungen speziell diese Substanz vorteilhaft umgesetzt werden soll. Unter vergleichbaren Bedingungen läßt nur der Einsatz von Ammoniak als Base in kurzer Reaktionszeit eine hohe Ausbeute an Thioglykolsäure erwarten.

Obwohl nun bereits eine ganze Reihe von Verfahren bekannt sind, wie man Thioglykolsäure aus Chloressigsäure herstellen kann, besteht noch ein Bedürfnis nach einem verbesserten, wirtschaftlich arbeitenden und die Belange der Umwelt berücksichtigenden Verfahren zur Herstellung von Thioglykolsäure.

Aufgabe der Erfindung ist es deshalb, ein Verfahren zur Verfügung zu stellen, das mit hohen Selektivitäten arbeitet, d.h. ein Verfahren, bei dem keine oder nur geringe Mengen an Nebenprodukten entstehen und bei dem sich keine anorganische Abfallprodukte wie Natriumchlorid oder Kaliumchlorid bilden.

Aufgabe der Erfindung ist es ferner ein Verfahren zur Verfügung zu stellen, das mit kurzen Reaktionszeiten auskommt und das sich insbesondere zur kontinuierlichen Durchführung eignet.

Diese Aufgabe wird gelöst durch ein Verfahren zur Herstellung von Thioglykolsäure durch Umsetzung von Monochloressigsäure mit Schwefelwasserstoff unter Druck in Gegenwart von tertiären Aminen in Lösung, wobei der Schwefelwasserstoffpartialdruck über dem Reaktionsmedium bis zum Reaktionsende mindestens 2 bar beträgt, und man die als Ammoniumsalz anfallende Thioglykolsäure mittels Säure freisetzt.

In vorteilhafter Weise kann der Schwefelwasserstoffpartialdruck durch eine Verbindung des Reaktionsraumes mit einer Quelle von unter Druck stehendem Schwefelwasserstoff während der Umsetzung aufrechterhalten werden.

Vorzugsweise liegt der Schwefelwasserstoffpartialdruck über 2 bar und in vorteilhafter Weise zwischen 10 bis 20 bar.

Die Reaktion kann vorteilhaft bei Raumtemperatur oder leicht erhöhten Temperaturen, d.h. bei Temperaturen von 15 bis 40°C durchgeführt werden.

In einer weiteren besonders vorteilhaften Ausführungsform des erfindungsgemäßen Verfahrens wird die Umsetzung von Anfang bis zum Ende unter einem konstanten, mindestens 2 bar betragendem Schwefelwasserstoffpartialdruck ausgeführt.

In einer weiteren besonders vorteilhaften Ausführungsform des erfindungsgemäßen Verfahrens wird als tertiäres Alkylamin Trimethylamin verwendet.

Es ist vorteilhaft, wenn man die Umsetzung in Gegenwart von etwa 2 bis 2,5 Mol tertiärem Amin pro Mol Monochloressigsäure durchführt. Sehr geeignet zur Umsetzung ist in Wasser gelöste Monochloressigsäure. Es ist vorteilhaft, wenn man die Umsetzung in homogener wäßriger Lösung durchführt, d.h. in der Weise, daß sowohl die Ausgangsstoffe als auch die sich bildenden Stoffe in Wasser gelöst bleiben. Vorzugsweise wird die Umsetzung dabei bei Temperaturen von 10 bis 60°C, insbesondere von 30 bis 40°C durchgeführt.

Die Umsetzung kann auch in einem organischen Lösungsmittel bzw. in einer Mischung von organischen Lösungsmitteln durchgeführt werden. Als geeignete Lösungsmittel seien beispielhaft genannt Methyl-t-butylether und Diisobutylketon.

Zur Freisetzung der Thioglykolsäure wird vorzugsweise Salzsäure verwendet.

Die Herstellung der Thioglykolsäure kann auf folgende Weise geschehen. Monochloressigsäure wird in Wasser gelöst, ferner wird separat eine Lösung aus Trimethylamin und Wasser zunächst mit Schwefelwasserstoff gesättigt und der gewünschte Schwefelwasserstoffpartialdruck eingestellt; dabei kann die Konzentration an Wasser in weiten Grenzen variieren. Bevorzugt wird eine Konzentration von Wasser, bezogen auf das Gesamtreaktionsgemisch von 15 bis 60 %. Besonders vorteilhaft sind Wasserkonzentrationen, die es erlauben, die Reaktion von Anfang bis zum Ende in homogener Phase, d.h. in Lösung durchzuführen, also eine Konzentration, bei der sowohl die Ausgangsstoffe als auch das entstehende Endprodukt löslich sind. Dieser Bereich liegt z.B. bei der Verwendung von Trimethylamin im Gebiet von etwa 40 bis 50 Gew.% Wasser.

Es versteht sich von selbst, daß die Konzentration des Wassers auch abhängig ist von dem verwendeten tertiären Amin und der Reaktionstemperatur.

Man kann also durch Sättigung unter Druck einen so hohen Schwefelwasserstoffpartialdruck einstellen, daß er während der gesamten Reaktionszeit mindestens 2 bar beträgt, ohne daß man während der Umsetzung zusätzlich weiteren frischen Schwefelwasserstoff in den Reaktionsraum einleitet.

In die mit Schwefelwasserstoff gesättigte und unter dem gewünschten Schwefelwasserstoffpartialdruck stehende Trimethylaminlösung wird dann die in Wasser gelöste Monochloressigsäure dosiert. Selbstverständlich ist es aber auch möglich, die wäßrige Trimethylaminlösung in eine unter Schwefelwasserstoff stehende Vorlage von Monochloressigsäure zu dosieren.

Es können grundsätzlich alle tertiären Amine verwendet werden, wobei kurzkettige tertiäre Alkylamine bevorzugt werden wie z.B. Triäthylamin.

Ganz besonders vorteilhaft ist der Einsatz von Trimethylamin, insbesondere deshalb, weil das bei der Umsetzung anfallende Trimethylaminhydrochlorid ein wertvolles Verkaufsprodukt ist.

Auch die Reaktionstemperatur kann in weiten Grenzen variiert werden und liegt vorteilhaft im Bereich von etwa 10 bis 60°C, selbstverständlich sind auch noch höhere oder niedere Temperaturen möglich. Besonders vorteilhaft ist der Temperaturbereich von 30 bis 40°C.

Das Verhältnis von Trimethylamin zu Monochloressigsäure sollte im allgemeinen mindestens 1,5 : 1 Mol betragen.

Vorzugsweise werden jedoch entsprechend der nachfolgenden Reaktionsgleichung stöchiometrische bzw. leicht überstöchiometrische Mengen an Rektanden eingesetzt, d.h. das Verhältnis tertiäres Amin zu Monochloressigsäure liegt bei 2 : 1 bis 2,5 : 1, vorzugsweise 2,1 bis 2,2 : 1.

$$Cl\text{-}CH_2\text{-}COOH + 2(CH_3)_3N + H_2S \rightarrow HS\text{-}CH_2\text{-}COO^-(CH_3)_3H\overset{\oplus}{N} + (CH_3)_3N \cdot HCl \qquad (1)$$

Eine weitere vorteilhafte Ausführungsform besteht darin, daß man während der Umsetzung dafür sorgt, daß das Reaktionsgefäß bzw. der Reaktionsraum in Verbindung mit einer unter überatmosphärischem Druck stehenden Schwefelwasserstoff-Quelle in Verbindung steht. Dies kann z.B. dadurch geschehen, daß ein Reaktionskessel über eine Zuführungsleitung in Verbindung mit einem Reservoir steht, in dem sich Schwefelwasserstoff unter überatmosphärischem Druck befindet. Man kann dabei den überatmosphärischen Schwefelwasserstoff oberhalb des Reaktionsmediums, insbesondere oberhalb der Reaktionsflüssigkeit einleiten, es ist jedoch auch möglich, über ein Rohr oder eine entsprechende Zuleitung den Schwefelwasserstoff mittels einer Düse einzuführen.

In einer besonders vorteilhaften Ausführungsform wird der überatmosphärische Schwefelwasserstoffpartialdruck während der gesamten Reaktionszeit konstant gehalten.

**Trennung der Reaktionsprodukte:**

Vorstehender Reaktionsgleichung zufolge liegt das gewünschte Produkt in Form seines Trimethylammoniumsalzes vor. Um die Thioglykolsäure als freie Säure zu gewinnen, wird das Reaktionsgemisch nach dem Entspannen des Reaktionsgefäßes mit einer entsprechenden Menge an Salzsäure versetzt, wobei freie Thioglykolsäure und ein zweites Mol Trimethylaminhydrochlorid entsteht. Die Thioglykolsäure läßt sich dann problemlos durch Extraktion von der wäßrigen Lösung abtrennen. Geeignete Extraktionsmittel sind Ether, Ketone, insbesondere Methyl-tert.-butylether MTBE, Methyl-tert.-Amylether TAME, Diisobutylketon. Im nächsten Trennungsschritt wird das Extraktionsmittel abdestilliert und kann damit erneut zur Extraktion eingesetzt werden. Der letzte Aufarbeitungsschritt besteht in einem Reinigungsschritt der Thioglykolsäure durch Destillation.

Die folgende Reaktionsgleichung gibt die Freisetzung des nach (1) entstandenen Thioglykolsäuresalzes wieder.

$$HS\text{-}CH_2\text{-}COO^{-}(CH_3)_3H\overset{\oplus}{N}+HCl \rightarrow HS\text{-}CH_2\text{-}COOH+(CH_3)_3N \cdot HCl \tag{2}$$

Es war besonders überraschend, daß bei dem Verfahren gemäß der Erfindung verhältnismäßig wenig Nebenprodukte entstehen, auch wenn man mit höheren Konzentrationen der Ausgangsstoffe arbeitet. Dies war nicht zu erwarten, weil bei den bekannten Reaktionen, bei dem ein Salz der Monochloressigsäure mit einem Hydrogensulfid, wie Natriumhydrogensulfid oder Kaliumhydrogensulfid, umgesetzt wird, bei steigenden Konzentrationen auch der Anteil an Nebenprodukten wie Thiodiglykol- und Dithiodiglykolsäure sehr stark steigt. Bei dem erfindungsgemäßen Verfahren hingegen bleibt die Selektivität auch in konzentrierter Lösung sehr hoch.

Es ist ferner nicht erforderlich, mit hohen Schwefelwasserstoffdrucken zu arbeiten. Es werden bereits mit niedrigeren $H_2S$-Drucken, z.B. 5 bar sehr hohe Selektivitäten erreicht.

Insbesondere beim Einsatz von Trimethylamin arbeitet das erfindungsgemäße Verfahren abfallfrei, d.h. es entstehen als Produkte Thioglykolsäure und Trimethylaminhydrochlorid, beides wertvolle Produkte, die direkt verwendet werden können. Das Verfahren arbeitet somit abfallfrei, wie aus der obigen Reaktionsgleichung ersichtlich ist.

Auch bei der Verwendung von anderen tertiären Aminen ist ohne weiteres eine Aufarbeitung möglich, ohne daß es dabei zu anorganischen Abfällen kommt, wie das bei dem konventionellen Verfahren der Fall ist, z.B. kann bei Verwendung von Trioctylamin das gebildete HCl-Salz thermisch in HCl-Gas und Amin gespalten werden.

Das Verfahren ermöglicht die Herstellung von Thioglykolsäure in hoher Reinheit, so daß das Produkt als solches direkt zum Einsatz gelangen kann bzw. direkt in die gewünschten Folgeprodukte, wie Salze oder Ester u.dgl., überführt werden kann.

Das erfindungsgemäße Verfahren ist besonders geeignet kontinuierlich durchgeführt zu werden.

Aufgrund der hohen Reaktionsgeschwindigkeit eignet sich dieses Verfahren auch hervorragend zur kontinuierlichen Prozeßführung in einer Kaskade von Rührkesselreaktoren. Das effiziente Reaktionssystem macht es möglich, daß hierbei nur wenige Rührkessel benötigt werden, um den vollständigen Umsatz der Monochloressigsäure zur Thioglykolsäure herbeizuführen.

Eingesetzt werden Stahlautoklaven, welche mit einer effektiven Rühreinrichtung und einer Druckkontroll- bzw. Druckregeleinrichtung versehen sind. Die Rührautoklaven sind so angeordnet, daß zunächst der vollständige Umsatz an Monochloressigsäure zum Trimethylammoniumsalz der Thioglykolsäure und anschließend die Freisetzung der Thioglykolsäure mit Salzsäure erfolgt. Die Produkttrennung erfolgt, wie oben beschrieben, ebenso kontinuierlich.

Die Erfindung wird durch folgende Beispiele näher erläutert:

**Beispiel 1**

In einem 1 Liter Büchi-Stahlautoklaven werden 216,7 g wäßrige Trimethylaminlösung vorgelegt, wobei der Trimethylamingehalt 45 % beträgt (1,65 Mol Trimethylamin). Diese Lösung wird dann mit Schwefelwasserstoff gesättigt, bis sich ein gewünschter Druck von 15 bar einstellt. Die Temperatur wird mit Hilfe eines Thermostaten auf 40°C eingestellt und die Vorlage mit einem Begasungsrührer bei 700 upm durchmischt. Die Reaktion beginnt, wenn die in 100 g Wasser gelösten 70,9 g Monochloressigsäure (0,75 Mol) innerhalb von einer Minute mit Hilfe einer Kolbenpumpe in das Reaktionsgefäß dosiert werden. Während der Reaktion werden Druck und Temperatur kontrolliert. Nach 10 Minuten ist die Monochloressigsäure vollkommen umgesetzt und es konnte eine Ausbeute an Thioglykolsäure von 96,0 % erzielt werden.

**Beispiel 2**

Ausführung wie in Beispiel 1, jedoch bei 20°C. Nach 30 Minuten ist die Monochloressigsäure vollkommen umgesetzt, die Ausbeute an Thioglykolsäure beträgt 97,5 %.

**Beispiel 3**

Ausführung wie in Beispiel 1, jedoch mit 207,8 g wäßriger Triethylaminlösung (152,0 g Triethylamin + 55,8 g Wasser). Nach 30 Minuten ist die Monochloressigsäure vollkommen umgesetzt und die Ausbeute an Thioglykolsäure beträgt 94,5 %.

**Beispiel 4**

Ausführung wie in Beispiel 1, jedoch bei 5 bar Schwefelwasserstoff. Nach 30 Minuten ist die Monochloressigsäure vollkommen umgesetzt, die Ausbeute an Thioglykolsäure beträgt 94,0 %.

**Beispiel 5**

Ausführung wie in Beispiel 1, jedoch nur mit 1,05 Mol Trimethylamin (Trimethylamin/Monochloressigsäure-Molverhältnis = 1,5 : 1). Die Monochloressigsäure ist nach 120 Minuten bei einer Ausbeute an Thioglykolsäure von 80 % vollkommen umgesetzt.

**Beispiel 6**

In einem 1 Liter Büchi-Stahlautoklaven werden 197 g wäßrige Trimethylaminlösung vorgelegt, wobei der Trimethylamingehalt 45 % beträgt. Diese Lösung wird dann mit Schwefelwasserstoff gesättigt, bis sich ein gewünschter Druck von 15 bar einstellt. Der Autoklav wird dann von der Schwefelwasserstoffversorgung abgetrennt. Die Temperatur wird mit Hilfe eines Thermostaten auf 30°C eingestellt und die Vorlage mit einem Begasungsrührer bei 700 upm durchmischt.

Die Reaktion beginnt, wenn die in 100 g Wasser gelösten 70,9 g Monochloressigsäure innerhalb von einer Minute mit Hilfe einer Kolbenpumpe in das Reaktionsgefäß dosiert werden. Durch die Dosierung der Monochloressigsäure wird ein Teil des in der Lösung gebundenen Schwefelwasserstoffs freigesetzt, wodurch der Druck im Reaktionsraum bis auf 25 bar ansteigt. Am Ende der Reaktion beträgt der Schwefelwasserstoffpartialdruck 23 bar.

Nach 30 Minuten ist die Monochloressigsäure vollkommen umgesetzt, und es konnte eine Ausbeute an Thioglykolsäure von 96,7 % erzielt werden.

**Beispiel 7**

In einem 1 Liter Büchi-Stahlautoklaven werden 192 g Methyl-tertiärer-butylether MTBE und 39 g wasserfreies Trimethylamin vorgelegt. Diese Lösung wird dann mit Schwefelwasserstoff gesättigt, bis sich ein gewünschter Druck von 10 bar einstellt. Die Temperatur wird mit Hilfe eines Thermostaten auf 20°C eingestellt und die Vorlage mit einem Begasungsrührer bei 700 upm durchmischt.

Die Reaktion beginnt, wenn die in 60 g Methyl-tertiärer-butylether MTBE gelösten 28,4 g Monochloressigsäure innerhalb von einer Minute mit Hilfe einer Kolbenpumpe in das Reaktionsgefäß dosiert werden. Nach 20 Minuten ist die Monochloressigsäure vollkommen umgesetzt und es konnte eine Ausbeute an Thioglykolsäure von 92,0 % erzielt werden.

**Beispiel 8**

Ausführung wie in Beispiel 7, jedoch anstelle von MTBE mit Diisobutylketon DIBK als Lösungsmittel. Nach 25 Minuten ist die Monochloressigsäure vollkommen umgesetzt, die Ausbeute an Thioglykolsäure beträgt 91 %.

**Patentansprüche**

1. Verfahren zur Herstellung von Thioglykolsäure durch Umsetzung von Monochloressigsäure mit Schwefelwasserstoff unter Druck in Gegenwart von tertiären Aminen in Lösung, wobei der Schwefelwasserstoffpartialdruck über dem Reaktionsmedium mindestens 2 bar beträgt, und man die als Ammoniumsalz anfallende Thioglykolsäure mittels Säure freisetzt.

2. Verfahren nach Anspruch 1, dadurch gekennzeichnet, daß der Schwefelwasserstoffpartialdruck durch eine Verbindung des Reaktionsraumes mit einer Quelle von unter Druck stehendem Schwefelwasserstoff während der Umsetzung aufrechterhalten wird.

3. Verfahren nach Anspruch 1 oder 2, dadurch gekennzeichnet, daß der Schwefelwasserstoffpartialdruck mehr als 2 bar beträgt.

4. Verfahren nach Anspruch 3, dadurch gekennzeichnet, daß der Schwefelwasserstoffpartialdruck 10 bis 20 bar beträgt.

5. Verfahren nach einem der Ansprüche 1 bis 4, dadurch gekennzeichnet, daß man die Umsetzung bei 15 bis 40°C durchführt.

6. Verfahren nach einem der Ansprüche 1 bis 5, dadurch gekennzeichnet, daß man die Umsetzung von Anfang bis zum Ende unter einem konstanten Schwefelwasserstoffpartialdruck ausführt.

7. Verfahren nach einem der Ansprüche 1 bis 6, dadurch gekennzeichnet, daß man tertiäre Alkylamine verwendet.

8. Verfahren nach Anspruch 7, dadurch gekennzeichnet, daß man als tertiäres Alkylamin Trimethylamin verwendet.

9. Verfahren nach einem der Ansprüche 1 bis 8, dadurch gekennzeichnet, daß man die Umsetzung in Gegenwart von 1,5 bis 2,5 holen tertiärem Amin pro Mol Monochloressigsäure durchführt.

10. Verfahren nach Anspruch 9, dadurch gekennzeichnet, daß man die Umsetzung in Gegenwart von 2,1 bis 2,2 holen tertiärem Amin pro Mol Monochloressigsäure durchführt.

11. Verfahren nach einem oder mehreren der Ansprüche 1 bis 10, dadurch gekennzeichnet, daß man zur Umsetzung wäßrige Monochloressigsäure verwendet.

12. Verfahren nach einem oder mehreren der Ansprüche 1 bis 11, dadurch gekennzeichnet, daß man die Umsetzung in homogener wäßriger Lösung durchführt.

13. Verfahren nach einem oder mehreren der Ansprüche 1 bis 10, dadurch gekennzeichnet, daß man die Umsetzung in einem organischen Lösungsmittel durchführt.

14. Verfahren nach Anspruch 13, dadurch gekennzeichnet, daß man die Umsetzung in einem Gemisch organischer Lösungsmittel durchführt.

15. Verfahren nach einem oder mehreren der Ansprüche 1 bis 14, dadurch gekennzeichnet, daß man die Umsetzung bei Temperaturen von 10 bis 60°C durchführt.

16. Verfahren nach Anspruch 15, dadurch gekennzeichnet, daß man die Umsetzung bei Temperaturen von 30 bis 40°C durchführt.

17. Verfahren nach einem der Ansprüche 1 bis 16, dadurch gekennzeichnet, daß man die Umsetzung bei einem Schwefelwasserstoffpartialdruck von 5 bis 40 bar durchführt.

18. Verfahren nach einem oder mehreren der Ansprüche 1 - 17, dadurch gekennzeichnet, daß man die Umsetzung kontinuierlich durchführt.

19. Verfahren nach einem oder mehreren der Ansprüch 1 bis 18, dadurch gekennzeichnet, daß man zur Freisetzung der Thioglykolsäure Salzsäure verwendet.

**Claims**

1. Process for the preparation of thioglycolic acid by reacting monochloroacetic acid with hydrogen sulfide under pressure in the presence of tertiary amines in solution, wherein the hydrogen sulfide partial pressure above the reaction medium is at least 2 bar and the thioglycolic acid which arises as an ammonium salt is liberated by means of acid.

2. Process according to Claim 1, characterised in that the hydrogen sulfide partial pressure is maintained during the reaction by connecting the reaction chamber to a source of pressurised hydrogen sulfide.

3. Process according to Claim I or 2, characterised in that the hydrogen sulfide partial pressure exceeds 2 bar.

4. Process according to Claim 3, characterised in that the hydrogen sulfide partial pressure is from 10 to 20 bar.

5. Process according to one of Claims 1 to 4, characterised in that the reaction is carried out at from 15 to 40°C.

6. Process according to one of Claims 1 to 5, characterised in that the reaction is carried out from start to finish at a constant hydrogen sulfide partial pressure.

7. Process according to one of Claims 1 to 6, characterised in that tertiary alkylamines are used.

8. Process according to Claim 7, characterised in that trimethylamine is used as a tertiary alkylamine.

9. Process according to one of Claims 1 to 8, characterised in that the reaction is carried out in the presence of from 1.5 to 2.5 moles tertiary amine per mole monochloroacetic acid.

10. Process according to Claim 9, characterised in that the reaction is carried out in the presence of from 2.1 to 2.2 moles tertiary amine per mole monochloroacetic acid.

11. Process according to one or more of Claims 1 to 10, characterised in that aqueous monochloroacetic acid is used for the reaction.

12. Process according to one or more of Claims 1 to 11, characterised in that the reaction is carried out in homogeneous aqueous solution.

13. Process according to one or more of Claims 1 to 10, characterised in that the reaction is carried out in an organic solvent.

14. Process according to Claim 13, characterised in that the reaction is carried out in a mixture of organic solvents.

15. Process according to one or more of Claims 1 to 14, characterised in that the reaction is carried out at temperatures of from 10 to 60°C.

16. Process according to Claim 15, characterised in that the reaction is carried out at temperatures of from 30 to 40°C.

17. Process according to one of Claims 1 to 16, characterised in that the reaction is carried out at a hydrogen sulfide partial pressure of from 5 to 40 bar.

18. Process according to one or more of Claims 1 to 17, characterised in that the reaction is carried out in continuous manner.

19. Process according to one or more of Claims 1 to 18, characterised in that hydrochloric acid is used to liberate the thioglycolic acid.

**Revendications**

1. Procédé de préparation d'acide thioglycolique par réaction d'acide monochloroacétique avec de l'hydrogène sulfuré sous pression, en présence d'amines tertiaires en solution, dans lequel la pression partielle d'hydrogène sulfuré au-dessus du milieu de réaction est d'au moins 2 bars, et l'acide thioglycolique produit sous la forme de sel d'ammonium est libéré au moyen d'un acide.

2. Procédé selon la revendication 1, caractérisé en ce que la pression partielle d'hydrogène sulfuré est maintenue pendant la réaction par mise en communication de l'enceinte de réaction avec une source d'hydrogène sulfuré sous pression.

3. Procédé selon la revendication 1 ou 2, caractérisé en ce que la pression partielle d'hydrogène sulfuré est de plus de 2 bars.

4. Procédé selon la revendication 3, caractérisé en ce que la pression partielle d'hydrogène sulfuré est de 10 à 20 bars.

5. Procédé selon l'une des revendications 1 à 4, caractérisé en ce que l'on conduit la réaction entre 15 et 40°C.

6. Procédé selon l'une des revendications 1 à 5, caractérisé en ce que l'on conduit la réaction sous une pression partielle d'hydrogène sulfuré constante du début à la fin.

7. Procédé selon l'une des revendications 1 à 6, caractérisé en ce que l'on utilise des alkylamines tertiaires.

8. Procédé selon la revendication 7, caractérisé en ce que l'on utilise comme alkylamine tertiaire la triméthylamine.

9. Procédé selon l'une des revendications 1 à 8, caractérisé en ce que l'on conduit la réaction en présence de 1,5 à 2,5 moles d'amine tertiaire par mole d'acide monochloroacétique.

10. Procédé selon la revendication 9, caractérisé en ce que l'on conduit la réaction en présence de 2,1 à 2,2 moles d'amine tertiaire par mole d'acide monochloroacétique.

11. Procédé selon l'une ou plusieurs des revendications 1 à 10, caractérisé en ce que, pour la réaction, on utilise de l'acide monochloroacétique en solution aqueuse.

12. Procédé selon l'une ou plusieurs des revendications 1 à 11, caractérisé en ce que l'on conduit la réaction dans une solution aqueuse homogène.

13. Procédé selon l'une ou plusieurs des revendications 1 à 10, caractérisé en ce que l'on réalise la réaction dans un solvant organique.

14. Procédé selon la revendication 13, caractérisé en ce que l'on réalise la réaction dans un mélange de solvants organiques.

15. Procédé selon l'une ou plusieurs des revendications 1 à 14, caractérisé en ce que l'on conduit la réaction à des températures de 10 à 60°C.

16. Procédé selon la revendication 15, caractérisé en ce que l'on conduit la réaction à des températures de 30 à 40°C.

17. Procédé selon l'une des revendications 1 à 16, caractérisé en ce que l'on conduit la réaction à une pression partielle d'hydrogène sulfuré de 5 à 40 bars.

18. Procédé selon l'une ou plusieurs des revendications 1-17, caractérisé en ce que l'on conduit la réaction en continu.

19. Procédé selon l'une ou plusieurs des revendications 1 à 18, caractérisé en ce que, pour la libération de l'acide thioglycolique, on utilise de l'acide chlorhydrique.